(19) Europäisches Patentamt
European Patent Office
Office européen des brevets

(11) Veröffentlichungsnummer: **0 006 449**
**B1**

(12) # EUROPÄISCHE PATENTSCHRIFT

(45) Veröffentlichungstag der Patentschrift: **08.12.82**

(51) Int. Cl.³: **C 07 D 221/26**, A 61 K 31/34, A 61 K 31/445, C 07 D 405/06

(21) Anmeldenummer: **79101583.7**

(22) Anmeldetag: **23.05.79**

(54) **2-(2-Alkoxyethyl)-2'-hydroxy-6,7-benzomorphane, deren Säureadditionssalze, diese enthaltende Arzneimittel und Verfahren zu deren Herstellung.**

(30) Priorität: **26.06.78 DE 2828039**

(43) Veröffentlichungstag der Anmeldung: **09.01.80 Patentblatt 80/1**

(45) Bekanntmachung des Hinweises auf die Patenterteilung: **08.12.82 Patentblatt 82/49**

(84) Benannte Vertragsstaaten: **AT BE CH DE FR GB IT LU NL SE**

(56) Entgegenhaltungen:
**DE-A-1 695 416**
**DE-A-2 002 864**
**DE-A-2 233 088**
**DE-A-2 411 382**
**DE-A-2 437 610**
**DE-A-2 606 267**
**DAZ, 1955, S. 1146**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, D-6507 Ingelheim am Rhein (DE)**

(72) Erfinder: **Merz, Herbert, Dr., Rotweinstrasse 53, D-6507 Ingelheim/Rhein (DE)**
Erfinder: **Stockhaus, Klaus, Dr., Pfarrer-Heberer-Strasse 35, D-6530 Bingen/Rhein (DE)**

**EP 0 006 449 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

2-(2-Alkoxyethyl)-2'-hydroxy-6,7-benzomorphane deren Säureadditionssalze, diese enthaltende Arzneimittel und Verfahren zu deren Herstellung

In der DE-A 2 233 088 werden 2-(2-Hydroxyalkyl)-5,9-dialkyl-2'-hydroxy-6,7-benzomorphane beschrieben, die analgetische und antitussive Wirksamkeit mit einer morphinantagonistischen Wirkungskomponente sowie mit sedierenden, muskelrelaxierenden und antidepressiven Eigenschaften in sich vereinigen.

Die DE-A 2 411 382 und DE-A 2 437 610 offenbaren 2-Tetrahydrofurfuryl-5,9-dialkyl-2'-hydroxy-6,7-benzomorphane denen gleichfalls gute analgetische Eigenschaften sowie Suchtfreiheit zugeschrieben werden.

Die Aufgabe der Erfindung besteht darin, neue Benzomorphan-Derivate mit verbesserten analgetischen Wirkungseigenschaften bereitzustellen.

Gegenstand der Erfindung sind 2-(2-Alkoxyethyl)-2'-hydroxy-6,7-benzomorphane der allgemeinen Formel

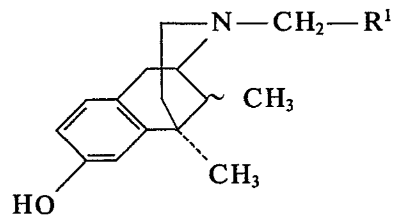

(I)

sowie die Säureadditionssalze dieser Verbindungen, diese enthaltende Arzneimittel und Verfahren zu deren Herstellung.

In der Formel bedeuten

$R^1$ (a) 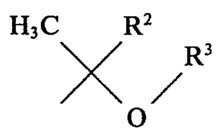 (b) 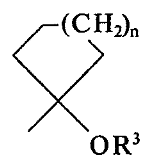 oder (c) 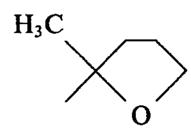

wobei

$R^2$ Wasserstoff oder Methyl,
$R^3$ Methyl, Ethyl, Propyl und
n die Zahl 1 oder 2 darstellen.

Bei der oben angegebenen Definition der Verbindungen der allgemeinen Formel I ergibt sich bezüglich der Stereochemie folgende Situation:

In dem den Verbindungen zugrunde liegenden 2'-Hydroxy-5,9-dimethyl-6,7-norbenzomorphan der Formel

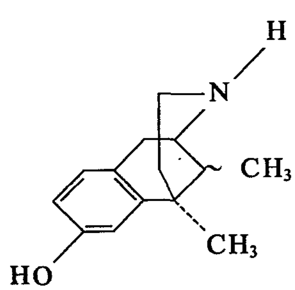

(II)

können die Methylgruppen bezüglich des carbocyclischen Ringes cis-ständig (5-Methyl-9α-methyl-6,7-benzomorphan) oder trans-ständig (5-Methyl-9β-methyl-6,7-benzomorphan) angeordnet sein. Sowohl in der α- als auch in der β-Reihe der Verbindung der Formel II existiert je ein Racemat mit den dazugehörigen optischen Antipoden.

Bei den N-Substituenten $R^1$ treten im Falle (a), wenn $R^2$ Wasserstoff bedeutet, und im Falle (c) chirale C-Atome in 2''-Position auf. Für diese Fälle verdoppelt sich die Anzahl der möglichen Stereoisomeren der allgemeinen Formel I.

Bevorzugt sind die linksdrehenden Verbindungen der Formel I. Besonders bevorzugt sind die (−)-Derivate, in denen $R^1$ eine 2''S-Konfiguration besitzt.

Die erfindungsgemäßen Verbindungen können durch Reduktion von 2-Acyl-2′-hydroxy-6,7-benzomorphanen der allgemeinen Formel

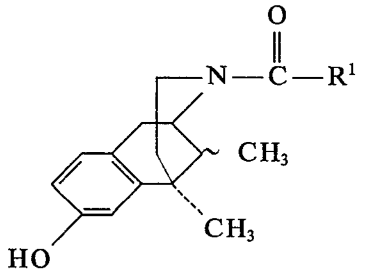

(III)

oder von 2-Acyl-2′-acyloxy-6,7-benzomorphanen der allgemeinen Formel

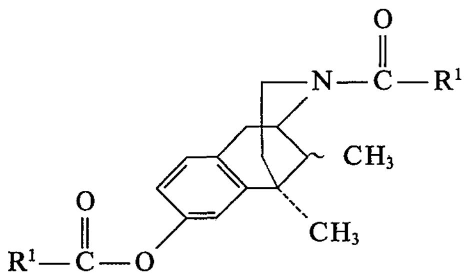

(IV)

worin $R^1$ die oben genannten Bedeutungen besitzt, hergestellt werden.

Für die Reduktion von Amiden der Formeln III und IV kommen verschiedene bekannte Methoden in Frage. Besonders bewährt hat sich die Verwendung von komplexen Hydriden als Reduktionsmittel, mit deren Hilfe sich die Reaktion einfach und zweckmäßig durchführen läßt. Obgleich verschiedene komplexe Hydride für diese Reaktion in Frage kommen, verwendet man vorzugsweise das leicht zugängliche Lithiumaluminiumhydrid. Das komplexe Hydrid wird zwecks vollständiger Umsetzung des relativ teuren Ausgangsproduktes der Formel III bzw. der Formel IV in der berechneten Menge oder vorzugsweise in einem Überschuß verwendet, wobei im allgemeinen ein Überschuß von 10 bis 50% genügt. Bei der Reduktion der Amide der Formel IV wird außer der Amidgruppierung auch die im allgemeinen leichter angreifbare Phenolestergruppierung reduziert. Die Reduktion wird vorteilhaft in einem geeigneten inerten Lösungsmittel oder Lösungsmittelgemisch vorgenommen. Bevorzugt werden zu diesem Zweck Diethyläther, Diisopropyläther und insbesondere Tetrahydrofuran. Die Reaktionstemperatur ist in weiten Grenzen variabel. Sie liegt vorteilhaft zwischen 0 und 150°C, vorzugsweise zwischen 20 und 75°C.

Die Aufarbeitung der Reaktionsprodukte sowie die Isolierung und Reindarstellung der gebildeten neuen Verbindungen der Formel I erfolgt nach bekannten Methoden. Gegebenenfalls können die erhaltenen Rohprodukte unter Anwendung besonderer Verfahren, z. B. der Säulenchromatographie gereinigt werden, ehe man sie in Form der Basen oder geeigneter Säureadditionsverbindungen kristallisiert.

Je nach Wahl der Reaktionsbedingungen und Reaktionspartner sind die gewonnenen Reaktionsprodukte entweder sterisch einheitliche Verbindungen oder Gemische aus racemisch bzw. optisch aktiven Diastereomeren.

Diastereomere können aufgrund ihrer unterschiedlichen chemischen und physikalischen Eigenschaften nach bekannten Verfahren z. B. durch fraktionierte Kristallisation getrennt werden. Racemische Verbindungen können mit Hilfe üblicher Methoden zur Racematspaltung in die entsprechenden optischen Antipoden getrennt werden.

Die Acylderivate der Formel III und der Formel IV erhält man durch Umsetzung eines Norbenzomorphans der Formel II mit einem Carbonsäurechlorid der Formel:

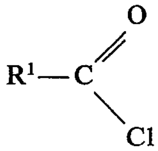

(V)

worin $R^1$ die oben genannte Bedeutung besitzt. Normalerweise werden die Ausgangsverbindungen der Formel III und IV aus Verbindungen der Formel II und V in situ dargestellt und ohne Reinigung weiter umgesetzt.

Die erfindungsgemäßen Verbindungen der allgemeinen Formel I sind Basen und können auf übliche Weise in ihre physiologisch verträglichen Säureadditionssalze überführt werden. Zur Salzbildung geeignete Säuren sind beispielsweise Mineralsäuren, wie Salzsäure, Bromwasserstoffsäure, Jodwasserstoffsäure, Fluorwasserstoffsäure, Schwefelsäure, Phosphorsäure, Salpetersäure oder organische Säuren wie Essigsäure, Propionsäure, Buttersäure, Valeriansäure, Pivalinsäure, Capronsäure, Oxalsäure, Malonsäure, Bernsteinsäure, Maleinsäure, Fumarsäure, Milchsäure, Brenztraubensäure, Weinsäure, Zitronensäure, Äpfelsäure, Benzoesäure, p-Hydroxybenzoesäure, Salicylsäure, p-Aminobenzoesäure, Phthalsäure, Zimtsäure, Ascorbinsäure, 8-Chlortheophyllin, Methansulfonsäure, Äthanphosphorsäure und dergleichen.

Die erfindungsgemäßen Verbindungen der Formel I und deren Säureadditionssalze üben eine therapeutisch nutzbare Wirkung auf das Zentralnervensystem aus. Besonders ausgeprägt ist die starke analgetische Wirkung, die in verschiedenen pharmakologischen Versuchsanordnungen an Laboratoriumstieren wie Mäusen und Ratten, z. B. im Writhing-Test oder Hot-Plate-Test demonstriert werden kann.

Die neuen Verbindungen übertreffen Morphin an Wirksamkeit um ein Vielfaches. Es fehlt jedoch das für morphinartige Analgetika typische Wirkungsbild, z. B. des Straub-Schwanz-phänomens und des Manegetriebs. Bei den Verbindungen, in denen der Rest $R^1$ die Bedeutung c) hat, tritt neben der Analgesie eine morphinantagonistische Wirkungskomponente auf. Das nicht morphinartige Wirkungsprofil der neuen Verbindungen der Formel I läßt darauf schließen, daß sie kein relevantes Mißbrauchspotential besitzen.

Die erfindungsgemäßen Verbindungen der allgemeinen Formel I sowie deren Säureadditionssalze können enteral oder auch parenteral angewandt werden. Die Dosierung für die enterale und parenterale Anwendung liegt bei etwa 0,5 bis 100 mg, vorzugsweise zwischen 1 und 20 mg. Die Verbindungen der Formel I bzw. deren Säureadditionssalze können mit anderen schmerzstillenden Mitteln oder mit andersartigen Wirkstoffen, z. B. Sedativa, Tranquilizern, Hypnotica kombiniert werden. Geeignete galenische Darreichungsformen sind beispielsweise Tabletten, Kapseln, Zäpfchen, Lösungen, Suspensionen, Pulver oder Emulsionen; hierbei können zu deren Herstellung die üblicherweise verwendeten galenischen Hilfs- und Trägerstoffe oder Spreng- oder Schmiermittel oder Substanzen zur Erzielung einer Depotwirkung Verwendung finden. Die Herstellung derartiger galenischer Darreichungsformen erfolgt auf übliche Weise nach den bekannten Fertigungsmethoden.

Die Tabletten können auch aus mehreren Schichten bestehen. Entsprechend können Dragées durch Überziehen von analog den Tabletten hergestellten Kernen mit üblicherweise in Dragéeüberzügen verwendeten Mitteln, beispielsweise Polyvinylpyrrolidon oder Schellack, Gummi arabicum, Talkum, Titandioxyd oder Zucker hergestellt werden.

Zur Erzielung eines Depoteffektes oder zur Vermeidung von Inkompatibilitäten kann der Kern auch aus mehreren Schichten bestehen. Desgleichen kann auch die Dragéehülle zur Erzielung einer Depotwirkung aus mehreren Schichten aufgebaut sein, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Zyklamat, Glycerin oder Zucker sowie ein geschmackverbesserndes Mittel, z. B. Aromastoffe wie Vanillin oder Orangenextrakt, enthalten. Sie können außerdem Suspendierungshilfsstoffe oder Dickungsmittel, wie Natriumcarboxymethylzellulose, Netzmittel, beispielsweise Kondensationsprodukte von Fettalkoholen mit Ethylendioxid oder Schutzstoffe, wie p-Hydroxybenzoate, enthalten.

Injektionslösungen werden in üblicher Weise, z. B. unter Zusatz von Konservierungsmitteln, wie p-Hydroxybenzoaten oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit mischt und Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyethylenglykol bzw. dessen Derivaten herstellen.

Die folgenden Beispiele erläutern die Erfindung in nicht beschränkter Weise.

## Herstellungsbeispiele

### Beispiel 1

### (–)-(1R, 5R, 9R, 2″R)-5,9-Dimethyl-2′-hydroxy-2-(2-methoxypropyl)-6,7-benzomorphan-hydrochlorid

a) (1R, 5R, 9R, 2″R)-5,9-Dimethyl-2′-hydroxy-2-(2-methoxypropionyl)-6,7-benzomorphan

2,17 g (0,01 Mol) (–)-5,9α-Dimethyl-2′-hydroxy-6,7-benzomorphan werden in 70 ml Methanol gelöst.

Die Lösung wird bei Raumtemperatur unter Rühren mit 2,5 g in 4 ml Wasser gelöstem Kaliumcarbonat und anschließend mit 1,5 g (0,0122 Mol) (+)-(R)-2-methoxy-propionylchlorid versetzt. Anschließend wird noch 2 Stunden bei Raumtemperatur weitergerührt und dann im Vakuum eingedampft. Der Eindampfungsrückstand wird mit 35 ml Chloroform und 15 ml Wasser geschüttelt, die abgetrennte wäßrige Phase noch einmal mit 10 ml Chloroform extrahiert. Die vereinigten Chloroformphasen werden nacheinander mit 10 ml 1 n HCl und 10 ml Wasser gewaschen, mit Natriumsulfat getrocknet und im Vakuum eingedampft. Der Rückstand besteht aus dem für die nachfolgende Reduktion benötigten Carbonsäureamiden.

b) (−)-(1R, 5R, 9R, 2″R)-5,9-Dimethyl-2′-hydroxy-2-(2-methoxy-propyl)-6,7-benzomorphan-hydrochlorid

Der Eindampfungsrückstand der vorausgehenden Reaktionsstufe wird in 25 ml absolutem Tetrahydrofuran gelöst und die Lösung innerhalb von 20 min unter Rühren zu einer mit Eiswasser gekühlten Suspension von 1,2 g (0,032 Mol) Lithiumaluminiumhydrid in 15 ml Tetrahydrofuran getropft. Anschließend wird das Eisbad entfernt, 1 Stunde bei Raumtemperatur weitergerührt und schließlich 2 Stunden unter Rückfluß gekocht. Darauf wird abgekühlt und die Reaktionsmischung unter Rühren und Kühlen mit Eiswasser tropfenweise mit 5 ml Wasser versetzt. Dann gibt man 120 ml gesättigte Diammoniumtartrat-Lösung zu, schüttelt im Scheidetrichter, trennt nach Absitzen die (obere) Tetrahydrofuranphase ab und extrahiert die wäßrige Phase mit 25 ml Chloroform. Der Eindampfungsrückstand der Tetrahydrofuran-Phase wird mit dem Chloroformextrakt vereinigt und die erhaltene Chloroformlösung mit Wasser gewaschen, mit Natriumsulfat getrocknet und im Vakuum eingedampft. Der Eindampfungsrückstand wird mit 10 ml Methanol gelöst, die Lösung mit 4,0 ml 2,5 n ethanolischer HCl und dann mit Ether versetzt, bis eben eine bleibende Trübung eintritt. Aus der Lösung kristallisiert die Titelverbindung. Zur Vervollständigung der Kristallisation läßt man über Nacht bei 0° C stehen. Dann wird abgesaugt, zuerst mit Methanol-Ether 1 : 1, dann mit Ether gewaschen und bei 80° C getrocknet. Ausbeute 1,8 g, 55,2% der Theorie, Schmelzpunkt 118° C. Umkristallisation aus 10 ml Methanol und 60 ml Ether gibt 1,4 g Substanz mit dem unveränderten Schmelzpunkt von 118° C und einer spezifischen Drehung von $/\alpha/_D^{22}$ = −83,8° (c = 1, Methanol).

Beispiel 2

(−)-(1R, 5R, 9R, 2″S)-5,9-Dimethyl-2′-hydroxy-2-(2-methoxy-propyl)-6,7-benzomorphan-hydrochlorid

Ausgehend von 2,17 g (0,01 Mol) (−)-5,9α-Dimethyl-2′-hydroxy-6,7-benzomorphan und 1,5 g (0,0122 Mol) (−)-(S)-2-Methoxypropionylchlorid erhält man analog Beispiel 1 die Titelverbindung in einer Ausbeute von 2,6 g = 79,8% der Theorie und mit einem Schmelzpunkt von 141° C.

Nach Umkristallisieren aus Methanol-Ether hat die Substanz den unveränderten Schmelzpunkt von 141° C und eine spezifische Drehung von $/\alpha/_D^{22}$ = −57,0° (c = 1, Methanol).

Beispiel 3

(+)-(1S, 5S, 9S, 2″R)-5,9-Dimethyl-2′-hydroxy-2-(2-methoxy-propyl)-6,7-benzomorphan-hydrochlorid

Ausgehend von 2,17 g (0,01 Mol) (+)-5,9α-Dimethyl-2′-hydroxy-6,7-benzomorphan und 1,5 g (0,0122 Mol) (+)-(R)-2-Methoxy-propionyl-chlorid erhält man analog Beispiel 1 die Titelverbindung in einer Ausbeute von 2,8 g = 85,9% der Theorie und mit einem Schmelzpunkt von 141° C. Nach Umkristallisieren aus Methanol-Ether schmilzt die Substanz unverändert bei 141° C und hat eine spezifische Drehung von $/\alpha/_D^{25}$ = +57,9° (c = 1, Methanol).

Beispiel 4

(−)-(1R, 5R, 9S, 2″R)-5,9-Dimethyl-2′-hydroxy-2-(2-methoxypropyl-6,7-benzomorphan-hydrochlorid

Ausgehend von 2,17 g (0,01 Mol) (−)-5,9β-Dimethyl-2′-hydroxy-6,7-benzomorphan und 1,5 g (0,0122 Mol) (+)-(R)-2-Methoxy-propionyl-chlorid erhält man analog Beispiel 1 die Titelverbindung in einer Ausbeute von 2,5 g = 76,7% der Theorie und mit einem Schmelzpunkt von 270° C. Nach Umkristallisieren aus Methanol-Ether schmilzt die Substanz unverändert bei 270° C und hat eine spezifische Drehung von $/\alpha/_D^{22}$ = −83,2° (c = 1, Methanol).

Beispiel 5
(−)-(1R, 5R, 9S, 2″S)-5,9-Dimethyl-2′-hydroxy-2-(2-methoxy-propyl)-6,7-benzomorphan-hydrochlorid

Ausgehend von 2,17 g (0,01 Mol) (−)-5,9β-Dimethyl-2′-hydroxy-6,7-benzomorphan und 1,5 g (0,0122 Mol) (−)-(S)-2-methoxy-propionyl-chlorid erhält man analog Beispiel 1 die Titelverbindung in einer Ausbeute von 2,4 g = 73,6% der Theorie und mit einem Schmelzpunkt von 254°C. Nach Umkristallisieren aus Methanol-Ether schmilzt die Substanz unverändert bei 254°C und hat eine spezifische Drehung von $/\alpha/_D^{22}$ = −56,5° (c = 1, Methanol).

Beispiel 6
(±)-5,9α-Dimethyl-2′-hydroxy-2-(2-methoxyisobutyl)-6,7-benzomorphan

2,17 g (0,01 Mol) (±)-5,9α-Dimethyl-2′-hydroxy-6,7-benzomorphan und 1,7 g (0,0125 Mol) 2-Methoxyisobutyrylchlorid werden analog Beispiel 1a) umgesetzt. Das Umsetzungsprodukt wird wie dort beschrieben isoliert und analog Beispiel 1b) reduziert. Das Reduktionsprodukt wird wie dort angegeben mit Chloroform extrahiert. Nach Waschen, Trocknen und Eindampfen der Extrakte erhält man einen Eindampfungsrückstand, der aus Aceton kristallisiert wird. Ausbeute 1,4 g = 46,1% der Theorie, Schmelzpunkt 160°C.

Beispiel 7
(−)-5,9α-Dimethyl-2′-hydroxy-2-(2-methoxyisobutyl)-6,7-benzomorphan

Ausgehend von 2,17 g (0,01 Mol) (−)-5,9α-Dimethyl-2′-hydroxy-6,7-benzomorphan und 1,7 g (0,0125 Mol) 2-Methoxyisobutyrylchlorid erhält man analog Beispiel 6 die Titelverbindung in einer Ausbeute von 1,4 g = 46,1% der Theorie und mit einem Schmelzpunkt von 86−88°C (aus Petroläther). Nach Umkristallisieren aus Methanol-Wasser schmilzt die Substanz bei 91−91,5°C. $/\alpha/_D^{25}$ = −114° (c = 1, Ethanol).

Beispiel 8
(±)-5,9β-Dimethyl-2′-hydroxy-2-(2-methoxyisobutyl)-6,7-benzomorphan-hydrochlorid

Ausgehend von 3,26 g (0,015 Mol) (±)-5,9β-Dimethyl-2′-hydroxy-6,7-benzomorphan und 2,55 g (0,0188 Mol) 2-Methoxyisobutyrylchlorid erhält man analog Beispiel 1 die Titelverbindung in einer Ausbeute von 4,0 g = 78,5% der Theorie und mit einem Schmelzpunkt von 226−228°C.

Beispiel 9
(−)-5,9β-Dimethyl-2′-hydroxy-2-(2-methoxyisobutyl)-6,7-benzomorphan-hydrochlorid

Ausgehend von 3,26 g (0,015 Mol) (−)-5,9β-Dimethyl-2′-hydroxy-6,7-benzomorphan und 2,55 g (0,0188 Mol) 2-Methoxyisobutyrylchlorid erhält man analog Beispiel 1 die Titelverbindung in einer Ausbeute von 4,0 g = 78,5% der Theorie und mit einem Schmelzpunkt von 226−227°C.

Beispiel 10
(±)-5,9α-Dimethyl-2-(2-ethoxyisobutyl)-2′-hydroxy-6,7-benzomorphan

Ausgehend von 2,17 g (0,01 Mol) (±)-5,9α-Dimethyl-2′-hydroxy-6,7-benzomorphan und 1,65 g (0,011 Mol) 2-Ethoxyisobutyrylchlorid erhält man analog Beispiel 6 die Titelverbindung in einer Ausbeute von 2,0 g = 63,0% der Theorie und mit einem Schmelzpunkt von 135−137°C (Methanol-Wasser).

Beispiel 11
(±)-5,9α-Dimethyl-2′-hydroxy-2-(2-n-propyloxyisobutyl)-6,7-benzomorphan-hydrochlorid

Ausgehend von 2,17 g (0,01 Mol) (±)-5,9α-Dimethyl-2′-hydroxy-6,7-benzomorphan und 1,8 g (0,011 Mol) 2-n-Propoxyisobutyrylchlorid erhält man analog Beispiel 1 die Titelverbindung in einer Ausbeute von 1,7 g = 46,2% der Theorie und mit einem Schmelzpunkt von 185−187°C.

Beispiel 12

(±)-5,9α-Dimethyl-2'-hydroxy-2-(2-isopropoxyisobutyl)-6,7-benzomorphan

Ausgehend von 2,17 g (0,01 Mol) (±)-5,9α-Dimethyl-2'-hydroxy-6,7-benzomorphan und 1,8 g (0,011 Mol) 2-Isopropoxyisobutyrylchlorid erhält man analog Beispiel 6 die Titelverbindung in einer Ausbeute von 2,0 g = 54,6% der Theorie und mit einem Schmelzpunkt von 123–125° C (Methanol-Wasser).

Beispiel 13

(±)-5,9α-Dimethyl-2'-hydroxy-2-(1-methoxy-1-cyclopentyl)-methyl-6,7-benzomorphan

Ausgehend von 2,17 g (0,01 Mol) (±)-5,9α-Dimethyl-2'-hydroxy-6,7-benzomorphan und 1,8 g (0,011 Mol) 1-Methoxycyclopentan-1-carbonsäurechlorid erhält man analog Beispiel 6 die Titelverbindung in einer Ausbeute von 16 g = 48,6% der Theorie und mit einem Schmelzpunkt von 88°C (Methanol-Wasser).

Beispiel 14

(–)-5,9α-Dimethyl-2'-hydroxy-2-(1-methoxy-1-cyclopentyl)-methyl-6,7-benzomorphan

Ausgehend von 2,17 g (0,01 Mol) (–)-5,9α-Dimethyl-2'-hydroxy-6,7-benzomorphan und 1,8 g (0,011 Mol) 1-Methoxycyclopentan-1-carbonsäurechlorid erhält man analog Beispiel 6 die Titelverbindung in einer Ausbeute von 1,8 g = 54,6% der Theorie und mit einem Schmelzpunkt von 88–90°C (Methanol-Wasser).

Beispiel 15

(±)-5,9β-Dimethyl-2'-hydroxy-2-(1-methoxy-1-cyclopentyl)-methyl-6,7-benzomorphan-hydrochlorid

Ausgehend von 2,17 g (0,01 Mol) (±)-5,9β-Dimethyl-2'-hydroxy-6,7-benzomorphan und 1,8 g (0,011 Mol) 1-Methoxycyclopentan-1-carbonsäurechlorid erhält man analog Beispiel 1 die Titelverbindung in einer Ausbeute von 3,0 g = 82,0% und mit einem Schmelzpunkt von 232–234° C.

Beispiel 16

(±)-5,9α-Dimethyl-2-(1-ethoxy-1-cyclopentyl)-methyl-2'-hydroxy-6,7-benzomorphan-hydrochlorid

Ausgehend von 2,17 g (0,01 Mol) (±)-5,9α-Dimethyl-2'-hydroxy-6,7-benzomorphan und 1,94 g (0,011 Mol) 1-Ethoxycyclopentan-1-carbonsäurechlorid erhält man analog Beispiel 1 die Titelverbindung in einer Ausbeute von 1,7 g = 44,7% und mit einem Schmelzpunkt von 200–202° C.

Beispiel 17

(±)-5,9β-Dimethyl-2-(1-ethoxy-1-cyclopentyl)-methyl-2'-hydroxy-6,7-benzomorphan-hydrochlorid

Ausgehend von 2,17 g (0,01 Mol) (±)-5,9β-Dimethyl-2'-hydroxy-6,7-benzomorphan und 1,94 g (0,011 Mol) 1-Ethoxycyclopentan-1-carbonsäurechlorid erhält man die Titelverbindung in einer Ausbeute von 2,2 g = 57,9% der Theorie und mit einem Schmelzpunkt von 208–210° C.

Beispiel 18

(±)-5,9α-Dimethyl-2'-hydroxy-2-(1-methoxy-1-cyclohexyl)-methyl-6,7-benzomorphan-hydrochlorid

Ausgehend von 2,17 g (0,01 Mol) (±)-5,9α-Dimethyl-2'-hydroxy-6,7-benzomorphan und 1,94 g (0,011 Mol) 1-Methoxycyclohexan-1-carbonsäurechlorid erhält man analog Beispiel 1 die Titelverbindung in einer Ausbeute von 1,3 g = 21,0% und mit einem Schmelzpunkt von 220° C.

Beispiel 19
(±)-5,9β-Dimethyl-2'-hydroxy-2-(1-methoxy-1-cyclohexyl)-methyl-6,7-benzomorphan-hydrochlorid

Ausgehend von 2,17 g (0,01 Mol) (±)-5,9β-Dimethyl-2'-hydroxy-6,7-benzomorphan und 1,94 g (0,011 Mol) 1-Methoxycyclohexan-1-carbonsäurechlorid erhält man analog Beispiel 1 die Titelverbindung in einer Ausbeute von 1,3 g = 34,2% und mit einem Schmelzpunkt von 267° C.

Beispiel 20
(−)-(1R, 5R, 9R, 2''R)-5,9-Dimethyl-2'-hydroxy-2-(2-methyl-tetrahydrofurfuryl)-6,7-benzomorphan-hydrochlorid und
(−)-(1R, 5R, 9R, 2''S)-5,9-Dimethyl-2'-hydroxy-2-(2-methyl-tetrahydrofurfuryl)-6,7-benzomorphan

a) Gemisch der beiden diastereomeren (−)-5,9α-Dimethyl-2'-hydroxy-2-(2-methyl-tetrahydro-2-furoyl)-6,7-benzomorphane (Amidzwischenstufe)

Eine Mischung aus 10,87 g (0,05 Mol) (−)-5,9α-Dimethyl-2'-hydroxy-6,7-benzomorphan und 7,81 g (0,06 Mol) racemischer 2-Methyl-tetrahydrofuran-2-carbonsäure wird mit 50 ml Methanol unter Erwärmen gelöst. Die Lösung wird im Stickstoffstrom im Ölbad bei 60° C eingedampft. Danach wird unter weiterem Überleiten eines trockenen Stickstoffstroms die Badtemperatur gesteigert und 8 Stunden bei 240° C gehalten. Nach Erkalten wird das Reaktionsprodukt in Chloroform gelöst und die Lösung nacheinander mit 2 n HCl und zweimal mit Wasser gewaschen. Eindampfen der mit Natriumsulfat getrockneten Chloroformlösung ergibt einen Eindampfungsrückstand, der aus einem Gemisch der diastereomeren Amidzwischenprodukte besteht.

b) (−)-(1R, 5R, 9R, 2''R)-5,9-Dimethyl-2'-hydroxy-2-(2-methyl-tetrahydrofurfuryl)-6,7-benzomorphan-hydrochlorid

Das in der vorausgehenden Reaktionsstufe erhaltene Amidgemisch wird analog Beispiel 1b) mit $LiAlH_4$ reduziert. Man erhält ein Reduktionsprodukt, das man in 25 ml Ethanol und 25 ml 2 n ethanolischer HCl löst. Die Lösung wird mit Ether bis eben zur beginnenden Trübung versetzt. Aus der Lösung scheidet sich die Titelverbindung kristallin ab. Nach 15 Stunden bei 0° C wird sie abgesaugt, mit Ethanol-Ether gewaschen und bei 80° C getrocknet. Ausbeute 1,8 g (10,2%, bezogen auf eingesetztes Benzomorphan), Schmelzpunkt 245° C (Zers.). Nach Umkristallisieren aus 20 ml Methanol und 80 ml Ether schmilzt die Substanz bei 276° C (nochmaliges Umkristallisieren ändert den Schmelzpunkt nicht mehr).
$/\alpha/_D^{25} = -77{,}1°$ (c=1,0, $H_2O$).

c) (−)-(1R, 5R, 9R, 2''S)-5,9-Dimethyl-2'-hydroxy-2-(2-methyl-tetrahydrofurfuryl)-6,7-benzomorphan

Die Mutterlauge der ersten Kristallisation des 2''R-Diastereomeren wird im Vakuum eingedampft und der Rückstand mit 100 ml Chloroform, 100 ml Wasser und 5 ml konz. Ammoniak geschüttelt. Nach Abtrennen der Chloroformphase wird diese mit Wasser gewaschen, mit Natriumsulfat getrocknet und im Vakuum eingedampft. Eine Lösung des Eindampfungsrückstandes in 20 ml Toluol, die man mit Petroläther (ca. 60 ml) verdünnt, scheidet Kristalle ab, die nach Stehen über Nacht im Kühlschrank abgesaugt, mit Toluol-Petroläther gewaschen und bei 80° C getrocknet werden. Man erhält die Titelverbindung in einer Ausbeute von 3,0 g = 19,0% der Theorie (bezogen auf eingesetztes Benzomorphan) und mit einem Schmelzpunkt von 140−141° C, der sich nach Umkristallisieren aus Toluol-Petroläther auf 142° C erhöht. $/\alpha/_D^{25} = -72{,}7°$ (Hydrochlorid, c=1,0, Wasser, hergestellt aus äquivalenten Mengen der Base und HCl).

Beispiel 21
(−)-(1R, 5R, 9R, 2''R)-5,9-Dimethyl-2'-hydroxy-2-(2-methyl-tetrahydrofurfuryl)-6,7-benzomorphan-hydrochlorid

Ausgehend von 1,09 g (0,005 Mol) (−)-5,9α-Dimethyl-2'-hydroxy-6,7-benzomorphan und 0,78 g (0,006 Mol) R-2-Methyl-tetrahydrofuran-2-carbonsäure erhält man analog Beispiel 20 die Titelverbindung in einer Ausbeute von 0,37 g = 21,0% der Theorie und mit einem Schmelzpunkt von 276° C (Zers.).

Beispiel 22
(−)-(1R, 5R, 9R, 2″S)-5,9-Dimethyl-2′-hydroxy-
2-(2-methyl-tetrahydrofurfuryl)-6,7-benzomorphan

Ausgehend von 1,09 g (0,005 Mol) (−)-5,9α-Dimethyl-2′-hydroxy-6,7-benzomorphan und 0,78 g (0,006 Mol) S-2-Methyl-tetrahydrofuran-2-carbonsäure erhält man durch Umsetzung analog Beispiel 20 und Kristallisation des Reaktionsprodukts aus Toluol-Petroläther die Titelverbindung in einer Ausbeute von 0,3 g = 19,0% der Theorie und mit einem Schmelzpunkt von 142° C.

Beispiel 23
(±)-(1R/S, 5R/S, 9R/S, 2″R/S)-5,9-Dimethyl-2′-hydroxy-2-(2-methyl-tetrahydrofurfuryl)-
6,7-benzomorphan-hydrochlorid und
(±)-(1R/S, 5R/S, 9R/S, 2″S/R)-5,9-Dimethyl-2′-hydroxy-2-
(2-methyl-tetrahydrofurfuryl)-6,7-benzomorphan

Durch Umsetzung von 10,87 g (0,05 Mol) (±)-5,9α-Dimethyl-2′-hydroxy-6,7-benzomorphan mit 7,81 g (0,06 Mol) racemischer 2-Methyl-tetrahydrofuran-2-carbonsäure erhält man analog Beispiel 20a) ein Gemisch der diastereomeren Amidzwischenprodukte, das analog Beispiel 20b) reduziert wird. Dabei erhält man ein Gemisch der diastereomeren Titelverbindung, das analog Beispiel 20b), c) aufgetrennt wird. Man erhält dabei die erste der beiden Titelverbindungen in einer Ausbeute von 1,9 g (10,8% der Theorie, bezogen auf das eingesetzte Benzomorphan), Schmelzpunkt 257° C, und die zweite der Titelverbindung in einer Ausbeute von 2,0 g (12,7%, bezogen auf das eingesetzte Benzomorphan, Schmelzpunkt 120 − 122, nach Umkristallisieren 128° C.

Beispiel 24
(−)-(1R, 5R, 9S, 2″R)-5,9-Dimethyl-2′-hydroxy-2-(2-methyl-tetrahydrofurfuryl)-
6,7-benzomorphan-hydrochlorid und
(−)-(1R, 5R, 9S, 2″S)-5,9-Dimethyl-2′-hydroxy-2-(2-methyl-tetrahydrofurfuryl)-
6,7-benzomorphan-hydrochlorid

Durch Umsetzung von 10,85 g (0,05 Mol) (−)-5,9β-Dimethyl-2′-hydroxy-6,7-benzomorphan mit 7,81 g (0,06 Mol) racemischer 2-Methyltetrahydrofuran-2-carbonsäure analog Beispiel 20a) erhält man ein Gemisch der diastereomeren Amidzwischenprodukte, das analog Beispiel 20b) reduziert wird. Das wie dort beschrieben isolierte Reduktionsprodukt besteht aus einem Gemisch der diastereomeren Titelverbindung, die folgendermaßen getrennt werden können:

a) Isolierung der (1R, 5R, 9S, 2″R)-Verbindung

Das Reduktionsprodukt scheidet aus einer salzsauren Lösung in Methanol-Ether Kristalle ab (5,5 g) die aus 70 ml Methanol und 200 ml Ether umkristallisiert werden. Die dabei erhaltene 4,2 g Substanz werden noch einmal aus 300 ml Ethanol umkristallisiert und ergeben 3,2 g noch nicht ganz reine Titelverbindung (Ausbeute 18,2% bezogen auf das eingesetzte Benzomorphan). Zur völligen Reinigung wird das Hydrochlorid (3,2 g) in die Base überführt und diese aus 20 ml Toluol kristallisiert. Man erhält 1,3 g Base, die unter Zusatz der berechneten Menge Methansulfonsäure in wenig Methonal gelöst wird. Aus der mit Ether versetzten Lösung kristallisiert das Methansulfonat (2,0 g, Schmelzpunkt 212° C), das aus 20 ml Methanol + 100 ml Ether umkristallisiert wird (1,7 g, Schmelzpunkt 212° C, dünnschichtchromatographisch rein).

Das Methansulfonat wird über die Base in das Hydrochlorid verwandelt, das aus Methanol-Ether kristallisiert. Man erhält dabei die völlig reine erste Titelverbindung: 1,5 g, Schmelzpunkt 279 − 280°; $/\alpha/_D^{25}$ = −73,9° (c = 1,0, Wasser).

b) Isolierung der (1R, 5R, 9S, 2″S)-Verbindung

Die unter a) beschriebene Abtrennung des ersten Diastereomeren (5,5 g) hinterbleibende Mutterlauge wird eingedampft und der Eindampfungsrückstand in die Base überführt. Diese wird aus 30 ml Toluol kristallisiert. Die dabei erhaltene Substanz (2,7 g, Schmelzpunkt 196 − 197° C) wird noch einmal aus Toluol (20 ml) kristallisiert, wobei man die reine Base in einer Ausbeute von 2,4 g (15,2% bezogen auf das eingesetzte Benzomorphan) und mit einem Schmelzpunkt von 197 − 198° C erhält. Die Base liefert aus einer mit ethanolischer Salzsäure angesäuerten Lösung in Methanol, die mit Ether bis eben zur Trübung versetzt wird, die zweite Titelverbindung in einer Ausbeute von 2,45 g und mit einem Schmelzpunkt von 265° C. $/\alpha/_D^{25}$ = −83,9° (c = 1,0, Wasser).

Beispiel 25

(±)-(1R/S, 5R/S, 9S/R, 2″S/R)-5,9-Dimethyl-2′-hydroxy-2-(2-methyl-tetrahydrofurfuryl)-6,7-benzomorphan-hydrochlorid und
(±)-1R/S, 5R/S, 9S/R, 2″R/S)-5,9-Dimethyl-2′-hydroxy-2-(2-methyl-tetrahydrofurfuryl)-6,7-benzomorphan-hydrochlorid

Durch Umsetzung von 10,85 g (0,05 Mol) (±)-5,9β-Dimethyl-2′-hydroxy-6,7-benzomorphan mit 7,81 g (0,06 Mol) racemischer 2-Methyl-tetrahydrofuran-2-carbonsäure analog Beispiel 20a) erhält man ein Gemisch der diastereomeren Amidzwischenprodukte, das analog Beispiel 20b) reduziert wird. Das wie dort beschriebene Reduktionsprodukt besteht aus einem Gemisch der diastereomeren Titelverbindung, die folgendermaßen getrennt werden können:

a) Isolierung der (1R/S, 5R/S, 9S/R, 2″S/R)-Verbindung

Das Reduktionsprodukt scheidet aus einer Lösung in 50 ml Petroläther 2,5 g kristalline Substanz aus, die bei 167°C schmilzt und nach Umkristallisieren aus 20 ml Toluol in völlig reiner Form erhalten wird (1,8 g, Schmelzpunkt 168°C). Aus der Mutterlauge des Erstkristallisats können noch weitere 0,7 g Substanz vom Schmelzpunkt 168°C gewonnen werden. Die Gesamtausbeute beträgt 2,5 g = 14,1% (bezogen auf das eingesetzte Benzomorphan). Die Base wird in Methanol unter Zugabe der berechneten Menge ethanolischer Salzsäure gelöst. Aus der mit Ether bis zur Trübung versetzten Lösung erhält man die erste Titelverbindung: 2,4 g, Schmelzpunkt 276–277°C.

b) Isolierung der (1R/S, 5R/S, 9S/R, 2″R/S)-Verbindung

Die Mutterlaugen der unter a) beschriebenen Kristallisation des ersten Diastereomeren als Base wird eingedampft und der Rückstand aus Methanol-Ether als Methansulfonat kristallisiert, das man in einer Ausbeute von 3,2 g und mit einem Schmelzpunkt von 224°C erhält. Aus 30 ml Methanol und 100 ml Ether umkristallisiert schmilzt dieses (2,7 g) bei 224°C. Das Methansulfonat wird über die Base in das Hydrochlorid überführt. Dieses kristallisiert aus seiner Lösung in Methanol-Ether in einer Ausbeute von 2,3 g (12,9%, bezogen auf das eingesetzte Benzomorphan) und mit einem Schmelzpunkt von 269°C.

Formulierungsbeispiele

Beispiel A

Tabletten

| | |
|---|---|
| Wirkstoff gemäß Erfindung | 20,0 mg |
| Milchzucker | 120,0 mg |
| Maisstärke | 50,0 mg |
| kolloidale Kieselsäure | 2,0 mg |
| lösliche Stärke | 5,0 mg |
| Magnesiumstearat | 3,0 mg |
| | 200,0 mg |

Herstellung:

Der Wirkstoff wird mit einem Teil der Hilfsstoffe gemischt und mit einer Lösung der löslichen Stärke in Wasser granuliert. Nach dem Trocknen des Granulats wird der Rest der Hilfsstoffe zugemischt und die Mischung zu Tabletten verpreßt.

Beispiel B

Dragées

| | |
|---|---|
| Wirkstoff gemäß Erfindung | 15,0 mg |
| Milchzucker | 100,0 mg |
| Maisstärke | 95,0 mg |
| kolloidale Kieselsäure | 2,0 mg |
| lösliche Stärke | 5,0 mg |
| Magnesiumstearat | 3,0 mg |
| | 220,0 mg |

Herstellung:
Der Wirkstoff und die Hilfsstoffe werden wie in Beispiel A beschrieben zu Tablettenkernen verpreßt, die mit Zucker, Talkum und Gummi arabicum üblicherweise dragiert werden.

Beispiel C

Suppositorien

| | |
|---|---|
| Wirkstoff gemäß Erfindung | 10,0 mg |
| Milchzucker | 150,0 mg |
| Suppositorienmasse q. s. ad | 1,7 g |

Herstellung:
Der Wirkstoff und der Milchzucker werden miteinander vermischt und die Mischung in der geschmolzenen Suppositorienmasse gleichmäßig suspendiert. Die Suspensionen werden in gekühlte Formen zu Suppositorien von 1,7 g Gewicht ausgegossen.

Beispiel D

Ampullen

| | |
|---|---|
| Wirkstoff gemäß Erfindung | 1,0 mg |
| Natriumchlorid | 10,0 mg |
| bidestilliertes Wasser q. s. ad | 1,0 ml |

Herstellung:
Der Wirkstoff und das Natriumchlorid werden in bidestilliertem Wasser gelöst und die Lösung in Ampullen steril abgefüllt.

Beispiel E

Tropfen

| | |
|---|---|
| Wirkstoff gemäß Erfindung | 0,70 g |
| p-Hydroxybenzoesäuremethylester | 0,07 g |
| p-Hydroxybenzoesäurepropylester | 0,03 g |
| entmineralisiertes Wasser q. s. ad | 100,00 ml |

Herstellung:
Der Wirkstoff und die Konservierungsmittel werden in demineralisiertem Wasser gelöst und die Lösung filtriert und in Flaschen zu je 100 ml abgefüllt.

**Patentansprüche für den Vertragsstaat AT**

1. Verfahren zur Herstellung von 2-(2-Alkoxyethyl)-2'-hydroxy-6,7-benzomorphanen der allgemeinen Formel

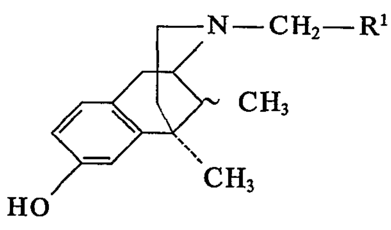

(I)

worin

$R^1$ (a) 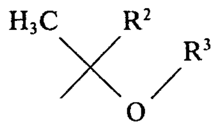 (b) 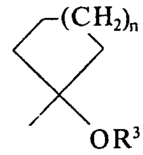 (c) 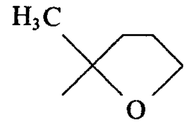

bedeuten, wobei

$R^2$ Wasserstoff oder Methyl,
$R^3$ Methyl, Ethyl, Propyl und
n die Zahl 1 oder 2 darstellen

sowie von deren Säureadditionssalzen, dadurch gekennzeichnet, daß man 2-Acyl-2'-hydroxy-6,7-benzomorphane der allgemeinen Formel

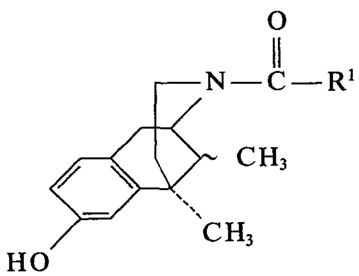

(II)

oder 2-Acyl-2'-acyloxy-6,7-benzomorphane der allgemeinen Formel

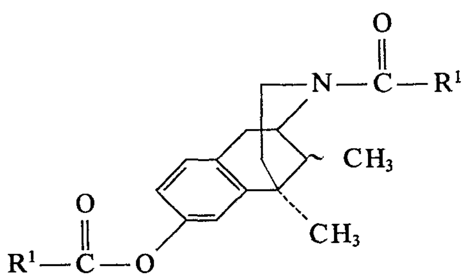

(III)

worin $R^1$ die oben genannten Bedeutungen hat, mit komplexen Metallhydriden reduziert und die erhaltenen Verbindungen gewünschtenfalls in ihre Säureadditionssalze überführt.

2. Verfahren nach Anspruch 1, dadurch gekennzeichnet, daß man Racemate oder racemische Gemische oder optisch aktive Formen der Ausgangsverbindungen der Formeln II oder III einsetzt.

3. Verfahren zur Herstellung von pharmazeutischen Zubereitungen, dadurch gekennzeichnet, daß man eine oder mehrere Verbindungen nach Anspruch 1 mit üblichen Hilfs- oder Trägerstoffen, Spreng- oder Schmiermitteln oder Substanzen zur Erzielung einer Depotwirkung zu pharmazeutischen Zubereitungen verarbeitet.

4. Verwendung von Verbindungen der allgemeinen Formel I nach Anspruch 1 zur Herstellung von Arzneimitteln für die Behandlung von Schmerzzuständen.

**Patentansprüche für die Vertragsstaaten BE, CH, DE, FR, GB, IT, LU, NL, SE**

1. 2-(2-Alkoxyethyl)-2'-hydroxy-6,7-benzomorphane der allgemeinen Formel

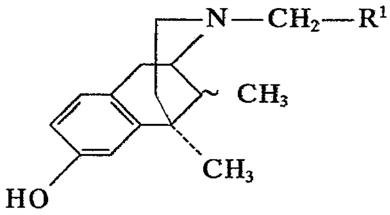

(I)

worin

$R^1$ (a) 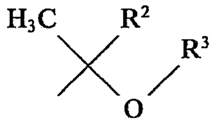 (b) 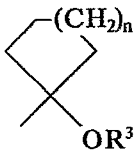 (c) 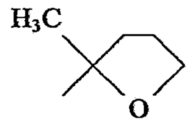

bedeuten, wobei

$R^2$ Wasserstoff oder Methyl,
$R^3$ Methyl, Ethyl, Propyl und
n die Zahl 1 oder 2 darstellen

sowie deren Säureadditionssalze.

2. Racemate oder racemische Gemische sowie optisch aktive Verbindungen nach Anspruch 1 und deren Säureadditionssalze.

3. Verfahren zur Herstellung von Verbindungen nach Anspruch 1, dadurch gekennzeichnet, daß man 2-Acyl-2'-hydroxy-6,7-benzomorphane der allgemeinen Formel

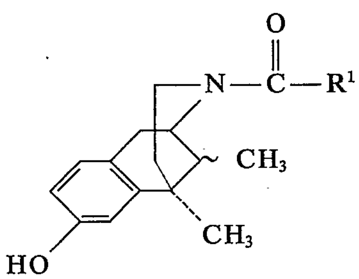

(II)

oder 2-Acyl-2'-acyloxy-6,7-benzomorphane der allgemeinen Formel

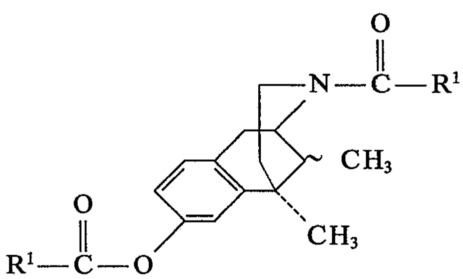

(III)

worin $R^1$ die in Anspruch 1 genannten Bedeutungen hat, mit komplexen Metallhydriden reduziert und die erhaltenen Verbindungen gewünschtenfalls in ihre Säureadditionssalze überführt.

4. Verfahren nach Anspruch 3, dadurch gekennzeichnet, daß man Racemate oder racemische Gemische oder optisch aktive Formen der Ausgangsverbindungen der Formeln II oder III einsetzt.

5. Pharmazeutische Zubereitungen, dadurch gekennzeichnet, daß sie als Wirkstoff eine oder mehrere Verbindungen nach Anspruch 1 enthalten.

6. Verfahren zur Herstellung von pharmazeutischen Zubereitungen nach Anspruch 5, dadurch gekennzeichnet, daß man eine oder mehrere Verbindungen nach Anspruch 1 mit üblichen Hilfs- oder Trägerstoffen, Spreng- oder Schmiermitteln oder Substanzen zur Erzielung einer Depotwirkung zu pharmazeutischen Zubereitungen nach Anspruch 5 verarbeitet.

7. Verbindungen der allgemeinen Formel I nach Anspruch 1 sowie deren Säureadditionssalze zur Verwendung bei der Behandlung von Schmerzzuständen.

**Claims for the contracting state AT**

1. Process for the preparation of 2-(2-alkoxyethyl)-2′-hydroxy-6,7-benzomorphanes of general formula

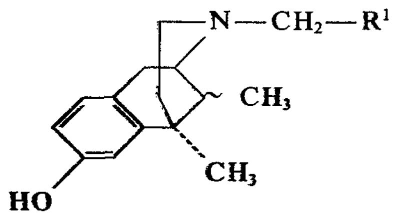

(I)

wherein

$R^1$ represents (a) 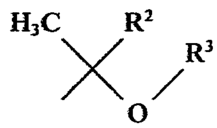 (b) 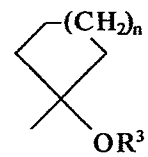 or (c) 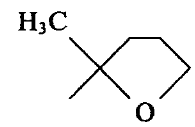

wherein

$R^2$ represents hydrogen or methyl,
$R^3$ represents methyl, ethyl or propyl, and
n represents the number 1 or 2,

and of the acid addition salts thereof, characterised in that 2-acyl-2′-hydroxy-6,7-benzomorphanes of general formula

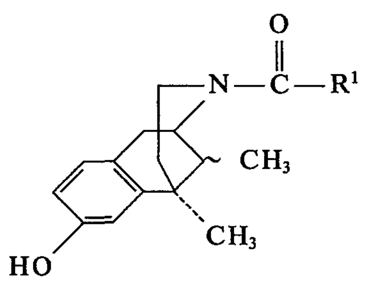

(II)

or 2-acyl-2′-acyloxy-6,7-benzomorphanes of general formula

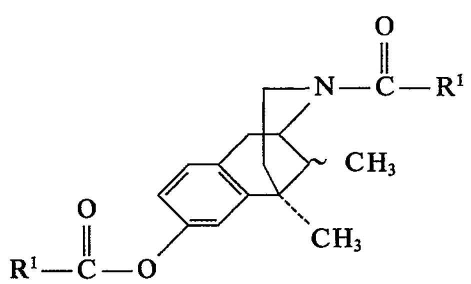

(III)

wherein $R^1$ is as hereinbefore defined, are reduced with complex metal hydrides and, if desired, the compounds obtained are converted into the acid addition salts thereof.

2. Process as claimed in claim 1, characterised in that racemates or racemic mixtures or optically active forms of the starting compounds of formula II or III are used.

3. Process for the preparation of pharmaceutical compositions, characterised in that one or more compounds according to claim 1 are processed with conventional excipients or carriers, disintegrants or lubricants or substances for obtaining delayed release, in order to produce pharmaceutical preparations.

4. Use of compounds of general formula I according to claim 1 for the preparation of pharmaceutical compositions for the treatment of pain.

**Claims for the contracting states BE, CH, DE, FR, GB, IT, LU, NL, SE**

1. 2-(2-Alkoxyethyl)-2′-hydroxy-6,7-benzomorphanes of general formula

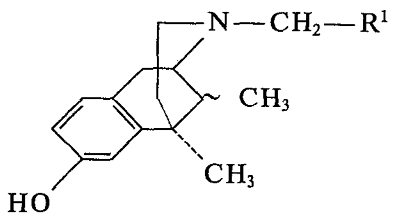

(I)

wherein

$R^1$ represents (a) 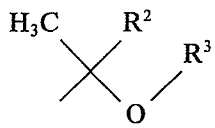 (b) 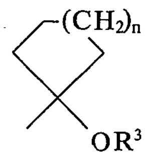 or (c) 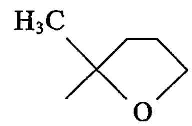

wherein

$R^2$ represents hydrogen or methyl,
$R^3$ represents methyl, ethyl or propyl, and
n represents the number 1 or 2,

and the acid addition salts thereof.

2. Racemates or racemic mixtures and optically active compounds according to claim 1 and the acid addition salts thereof.

3. Process for the preparation of compounds as claimed in claim 1, characterised in that 2-acyl-2′-hydroxy-6,7-benzomorphanes of general formula

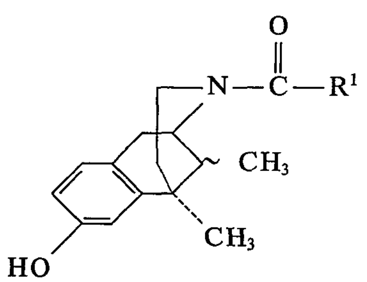

(II)

or 2-acyl-2′-acyloxy-6,7-benzomorphanes of general formula

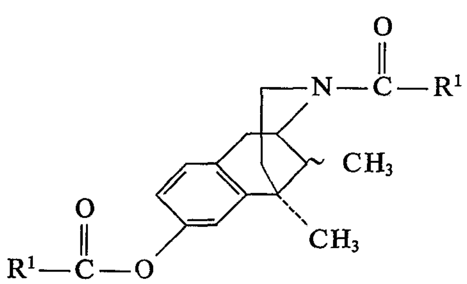

(III)

wherein $R^1$ is defined as in claim 1, are reduced with complex metal hydrides and, if desired, the compounds obtained are converted into the acid addition salts thereof.

4. Process as claimed in claim 3, characterised in that racemates or racemic mixtures or optically active forms of the starting compounds of formula II or III are used.

5. Pharmaceutical preparations, characterised in that they contain, as active substance, one or more compounds as claimed in claim 1.

6. Process for the preparation of pharmaceutical compositions as claimed in claim 5, characterised in that one or more compounds according to claim 1 are processed with conventional excipients or

carriers, disintegrants or lubricants or substances for obtaining delayed release, in order to produce pharmaceutical preparations according to claim 5.

7. Compounds of general formula I according to claim 1 and the acid addition salts thereof, for use in the treatment of pain.

**Revendications pour les Etats contractants: BE, CH, DE, FR, GE, IT, LU, NL, SE**

1. 2-(2-alcoxyéthyl)-2'-hydroxy-6,7-benzomorphanes de formule générale

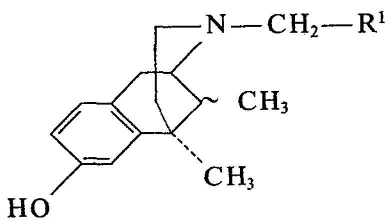

(I)

dans laquelle

$R^1$ signifie (a) 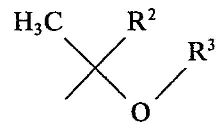 (b) 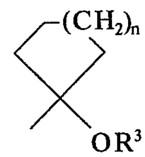 (c) 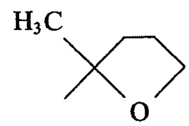

$R^2$ représentant hydrogène ou méthyle,
$R^3$ représentant méthyle, éthyle, propyle et
n représentant le nombre 1 ou 2

ainsi que leurs sels d'addition avec des acides.

2. Racémates ou mélanges racémiques ainsi que composés optiquement actifs selon la revendication 1 et leurs sels d'addition avec des acides.

3. Procédé pour la préparation des composés selon la revendication 1, caractérisé en ce qu'on réduit, au moyen d'hydrures métalliques complexes, des 2-acyl-2'-hydroxy-6,7-benzomorphanes de formule générale

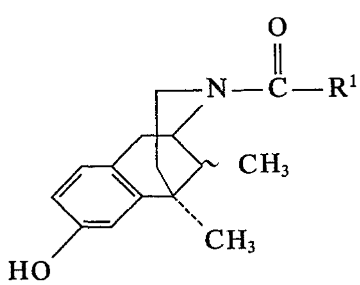

(II)

ou des 2-acyl-2'-acyloxy-6,7-benzomorphanes de formule générale

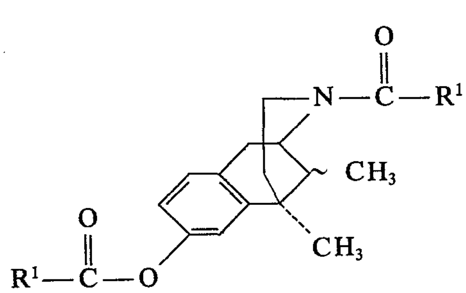

(III)

dans laquelle $R^1$ a les significations mentionnées à la revendication 1 et en ce qu'on transforme, si on le désire, les composés obtenus en leurs sels d'addition avec des acides.

4. Procédé selon la revendication 3, caractérisé en ce qu'on utilise des racémates ou des mélanges racémiques ou des formes optiquement actives des composés de départ de formule II ou III.

5. Préparations pharmaceutiques, caractérisées en ce qu'elles contiennent, en tant que substance active, un ou plusieurs composés selon la revendication 1.

6. Procédé pour la fabrication de préparations pharmaceutiques selon la revendication 5, caractérisé en ce qu'on transforme un ou plusieurs composés selon la revendication 1, en préparations pharmaceutiques selon la revendication 5, au moyen d'adjuvants ou excipients, de désagrégeants ou lubrifiants ou de substances pour obtenir un effet retard habituels.

7. Composés de formule générale I selon la revendication 1 ainsi que leurs sels d'addition avec des acides pour l'utilisation dans le traitement des états douloureux.

**Revendications pour l'Etat contractant: AT**

1. Procédé pour la préparation de 2(2-alcoxyéthyl)-2'-hydroxy-6,7-benzomorphanes de formule générale

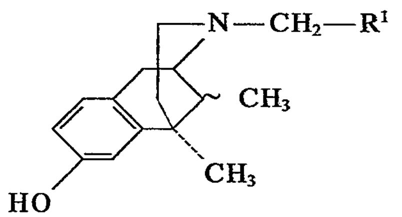

(I)

dans laquelle

$R^1$ signifie (a) 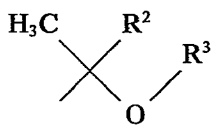 (b) 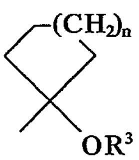 (c) 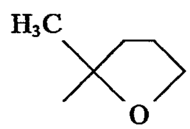

$R^2$ représentant hydrogène ou méthyle,
$R^3$ représentant méthyle, éthyle, propyle et
n représentant le nombre 1 ou 2

ainsi que leurs sels d'addition d'acides, caractérisé en ce qu'on réduit, au moyen d'hydrures métalliques complexes, des 2-acyl-2'-hydroxy-6,7-benzomorphanes de formule générale

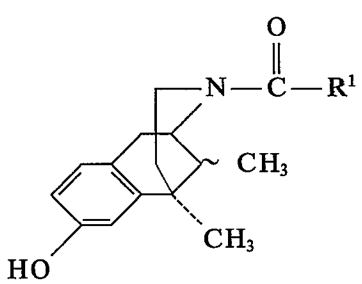

(II)

ou des 2-acyl-2'-acyloxy-6,7-benzomorphanes de formule générale

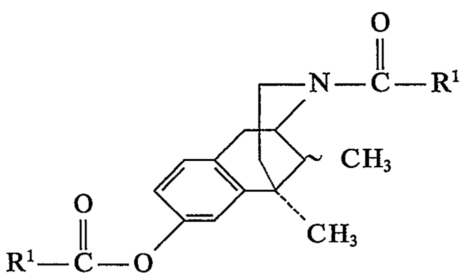

(III)

dans laquelle $R^1$ a les significations mentionnées plus haut et en ce qu'on transforme, si on le désire, les composés obtenus en leurs sels d'addition avec des acides.

2. Procédé selon la revendication 1, caractérisé en ce qu'on utilise des racémates ou des mélanges racémiques ou des formes optiquement actives des composés de départ de formule II ou III.

3. Procédé pour la fabrication de préparations pharmaceutiques, caractérisé en ce qu'on transforme un ou plusieurs composés selon la revendication 1 en préparations pharmaceutiques, au moyen d'adjuvants ou excipients, de désagrégeants ou lubrifiants ou de substances pour obtenir un effet retard habituels.

4. Utilisation des composés de formule générale I selon la revendication 1 pour la préparation de médicaments pour le traitement des états douloureux.